(19) Europäisches Patentamt
European Patent Office
Office européen des brevets

(11) EP 4 606 437 A1

(12) EUROPEAN PATENT APPLICATION

(43) Date of publication:
**27.08.2025 Bulletin 2025/35**

(21) Application number: **24315059.6**

(22) Date of filing: **23.02.2024**

(51) International Patent Classification (IPC):
***A61Q 19/08*** *(2006.01)* ***A61Q 5/00*** *(2006.01)*
***A61K 8/34*** *(2006.01)* ***A61K 8/35*** *(2006.01)*
***A61K 8/365*** *(2006.01)* ***A61K 8/49*** *(2006.01)*
***A61K 8/9789*** *(2017.01)*

(52) Cooperative Patent Classification (CPC):
**A61Q 5/00; A61K 8/347; A61K 8/35; A61K 8/365; A61K 8/498; A61K 8/9789; A61Q 19/08**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicants:
- **Laboratoire Native**
  **75008 Paris (FR)**
- **Centre National de la Recherche Scientifique (CNRS)**
  **75794 Paris Cedex 16 (FR)**
- **Université Claude Bernard Lyon 1 (UCBL)**
  **69616 Villeurbanne Cedex (FR)**

(72) Inventors:
- **ROUSSELLE, Patricia**
  **69005 Lyon (FR)**
- **DAYAN, Guila**
  **69100 Villeurbanne (FR)**
- **HAJEM-PACE, Neïla**
  **78290 Croissy-sur-Seine (FR)**
- **BARRIERE, Nicolas Alexandre**
  **92410 Ville d'Avray (FR)**
- **MANISSIER, Patricia**
  **92300 Levallois Perret (FR)**

(74) Representative: **Touroude, Magali Linda**
**Yes My Patent**
**32 Boulevard Richard Lenoir**
**75011 Paris (FR)**

# (54) COSMETIC COMPOSITIONS TO INHIBIT THE ACTIVITY OF KLF4, TO REDUCE SKIN, HAIR, MUCOUS MEMBRANES AGING

(57) The present invention concerns cosmetic compositions to inhibit the activity of KLF4, for skin anti-aging, especially by diminishing or preventing the consequences promoted by the stiffness of the extracellular matrix (ECM) networks located at the epithelial-stromal interfaces of tissues of the skin. Preferably, said inhibition of KLF4 is achieved through at least one compounds among phenylpropanoids, flavonoids, lignans, phenols, benzophenone derivative, homoisoflavonoids, stilbenoids, alkaloids, diterpenoids, phloracetophenone glucoside, polyketides, diarylheptanoids and/or adlerol.

EP 4 606 437 A1

## Description

The present invention concerns cosmetic compositions to inhibit the activity of KLF4, for skin anti-aging, especially by diminishing or preventing the consequences promoted by the stiffness of the extracellular matrix (ECM) networks located at the epithelial-stromal interfaces of tissues of the skin. Preferably, said inhibition of KLF4 is achieved through at least one compounds among phenylpropanoids, flavonoids, lignans, phenols, benzophenone derivative, homoisoflavonoids, stilbenoids, alkaloids, diterpenoids, phloracetophenone glucoside, polyketides, diarylheptanoids and/or adlerol.

## Technical Field

This invention is in the field of cosmetic and topical anti-aging compositions.

## Background Art

Basement membranes (BMs) are highly organized and well-defined planar extracellular matrix (ECM) networks located at the epithelial-stromal interfaces of tissues. They provide a structural support to cells and regulate their behavior (Yurchenco, 2011). The model of BM describes networks of polymerized laminins (LMs) and cross-linked collagen IV connected to one another by the glycoprotein, nidogen, and the heparan sulfate (HS) proteoglycan perlecan (Breitkreutz et al., 2013). BMs can serve as signaling platforms by sequestering growth factors thereby facilitating spatiotemporal regulation of receptor-ligand interactions (Jayadev & Sherwood, 2017). The structural organization and hydration state of ECM can have a direct impact on their mechanical properties and their capacity to generate mechanical signals to neighboring cells via a process that evolves during aging (Candiello et al., 2010; Pastor-Pareja & Xu, .2011).

In skin, the BM that connects the outer epidermis to the underlying dermis provides essential structural support for basal keratinocytes (Rousselle et al., 2022). Several adhesion structures form at this interface, including hemidesmosomes (HDs) that connect the BM to the intermediate filament of the cell cytoplasm via the integrin, $\alpha6\beta4$. Other structures at this interface include focal adhesions and epithelial podosomes, including $\beta1$-integrins that are connected to the actin cytoskeleton (Michopoulou et al., 2020; Watt, 2002). These adhesion structures are components of the dermal-epidermal junction (DEJ) and represent a specific informative niche that mediates signaling and epidermal renewal via the proliferation of stem cells and the differentiation of their progeny (Koster & Roop, 2007). In addition to the archetypal BM molecular network, the DEJ contains anchoring complexes with components that, when assembled, serve to increase the resistance of the epithelium to applied forces. These anchoring complexes connect basal keratinocytes, span the BM, and anchor the papillary dermis via the orderly assembly of specific factors including $\alpha6\beta4$-integrin, the LM isoform 332 (LM-332), and collagens VII and XVII (Has & Nyström, 2015; Te Molder et al., 2021).

The DEJ in human skin exhibit a characteristic microarchitecture with an undulating pattern generated by epidermal rete ridges, which are outgrowths of the epidermis that protrude into the dermis (Lawlor & Kaur, 2015). Rete ridges surround the dermal papillae, strengthen the connection between the dermis and epidermis, and improve the mechanical properties of the skin (Langton et al., 2017). Their lengths vary according to their anatomical location; this property appears to correlate positively with the force of the mechanical stresses to which a given tissue is subjected (Topczewska et al., 2019). Interestingly, the development of bioinspired skin-like scaffolds with microstructured waves that mimic the configuration of DEJ was found to promote the differentiation and stratification of reconstructed epithelia (Bush & Pins, 2012). When applied *in vivo,* these scaffolds can improve the re-epithelialization of wounds (Malara et al., 2020). These findings suggest that these topographically-patterned environments can communicate to resident cells and instruct them as to how they might respond to mechanical stress. At this time, the preferred position of human interfollicular epidermal stem cells (IFE-SCs) along the DEJ remains controversial. While several groups have identified IFE-SCs at the bases of the rete ridges, others have found them above the tip of the dermal papillae (Jones et al., 1995; Webb et al., 2004; Yamada et al., 2018). This discrepancy may be due to the heterogeneity of the cell population in the basal layer of the epidermis, as recently shown in a single-cell RNA sequencing study that targeted interfollicular cells of the human neonatal epidermis (S. Wang et al., 2020). Scaffolds mimicking the undulated geography of the DEJ promoted *in vitro* epidermization (Fu et al., 2014) and revealed the accumulation of bright-staining $\beta1$-integrin-positive keratinocytes at the tips of their domed structures via a mechanoresponsive mechanism. These findings support the hypothesis that stem keratinocytes localize at the uppermost edges of the rete ridges (Viswanathan et al., 2016).

As in all BMs, the DEJ thickens with aging due to alterations in its molecular composition and defects in its assembly (Khalilgharibi & Mao, 2021). The age-associated structural and biomechanical changes resulting from crosslinks in ECM networks induced by advanced glycation end products lead to increased stiffness of the BM and its underlying dermis (Candiello et al., 2010; Ewald, 2020). Aging skin frequently exhibits DEJ flattening due to the progressive loss of rete ridges and dermal papillae (Neerken et al., 2004; Newton et al., 2015). This altered topology leads to reduced contact between the epidermis and dermis which impairs two-way exchange and weakens epidermal anchorage and resistance to

mechanical shear forces (Farage et al., 2009). ECM stiffness determines cell behavior and gene expression via mechanisms involving integrin sensing and mechanotransduction (Engler et al., 2006; Janmey et al., 2020).

Based on the knowledge presented above, the Applicant surprisingly characterized that the age-related progressive increase in the stiffness of the dermal ECM has a profound influence on HD-mediated adhesion of basal keratinocytes and promotes their phenotypic change, leading to the loss of keratinocytes clusters with large HDs characteristic of progenitor cell niches and BM proteins required for HD formation and stability. Additional experiments performed *in vitro* show that increasing in stiffness disrupts HD-mediated adhesion and accelerates the entry of basal cells into the differentiation program. In addition, the Applicant surprisingly characterized a causal relationship between Krüppel-like factor 4 (KLF4) expression and the increasing stiffness of the ECM. The applicant also demonstrated for the first time that KLF4 expression sequentially and significantly increases in the epidermis during aging which is evidence of the loss of equilibrium between progenitor cells and differentiated cells because we know that the activation of KLF4 can lead to the inhibition of transcriptional networks that have an essential function in maintaining keratinocytes in their basal and undifferentiated state (Elbediwy et al., 2016; Yuan et al., 2020)..

Based on these findings, the Applicant developed skin and mucous membranes aging compositions based on the inhibition of this protein, characterizing a totally new pattern of addressing skin and mucous membranes aging.

The inhibition of the activity of KLF4 is the general common inventive concept of this invention. In particular, this inhibition provides a cosmetic antiaging efficacy.

## Disclosure of the invention

In a first aspect, the invention concerns a cosmetic non-therapeutic composition to inhibit the activity of Kruppel-like factor 4 (KLF4).

In another aspect, the invention concerns a cosmetic non-therapeutic composition comprising at least one compound to inhibit the activity of Kruppel-like factor 4 (KLF4).

By "inhibit the activity of KLF4" is intended according to the invention that the activity of KLF4 is less important, and is partially or totally inhibited. This inhibition ranges from 1% to 100%, preferably from 1% to 70%, more preferably from 1% to 50%.

In another aspect, the invention concerns a non-therapeutic cosmetic process for skin and/or scalp and/or hair and/or mucous membranes anti-aging care, comprising the topical application of a composition according to the invention.

In another aspect, the invention concerns the use of a cosmetic non-therapeutic composition to inhibit the activity of KLF4.

Preferably, said cosmetic uses and compositions are for skin anti-aging and/or scalp and/or hair and/or mucous membranes.

Preferably, said cosmetic uses and compositions are for diminishing or preventing the basal cell-adhesion consequences of stiffness of the extracellular matrix (ECM) networks located at the epithelial-stromal interfaces of tissues of the skin and/or scalp and/or hair and/or mucous membranes.

The Kruppel-like factor 4 (KLF4) protein acts as a transcription factor and is present in many cells of the human body, but particularly in the colon, esophagus, stomach and skin. One of the characteristics of the protein, like any protein interacting with DNA or RNA, is a section called zinc fingers. The zinc finger is a small protein structural motif that is characterized by the coordination of a Zinc ion enabling the stabilization of a specific $\alpha$-helix and anti-parallel $\beta$-sheet architecture. The KLF4 protein contains three C2H2 zinc fingers relatively close to its C-terminus.

The protein arginine N-methyltransferase 5 (PRMT5) is able to negatively modulate (Hu D. & Cie, Nat Commun. 2015 Sep ; 6, 8419. doi : 10.1038/ncomms9419) the KLF4 receptor. As this enzyme is essential for KLF4 methylation, its inhibition makes the protein more susceptible to degradation. PRMT5 is an enzyme involved in post-translational modifications (PTMs) and catalyzes several types of methylation (Cell Stress, Vol. 4, No. 8, pp. 199 - 215; doi: 10.15698/cst2020.08.228). The enzyme is present in many cells of the human body and notably in the same cells as the KLF4 protein.

Data from the Applicant (Example 1) showed that the expression of KLF4 was more important in samples donors of increasing age, as a result of an increasing stiffness of the ECM. By inhibiting the expression of KLF4, the compositions and uses according to the invention are diminishing or preventing the epidermal consequences of the increased stiffness of the ECM.

The technical effect of the invention is achieved by compounds bonding to KLF4, or bonding to its coactivator PRMT5, or inhibiting the interaction between KLF4 and PRMT5.

Related to all aspects of the invention, in an embodiment, said at least one compound in a cosmetically acceptable medium is at least one compound chosen among phenylpropanoids, flavonoids, lignans, phenols, benzophenone derivative, homoisoflavonoids, stilbenoids, alkaloids, diterpenoids, phloracetophenone glucoside, polyketides, diarylheptanoids and/or adlerol.

Alkaloids are a class of naturally occurring organic compounds that predominantly contain basic nitrogen atoms.

They are often found in plants and can exhibit a wide range of pharmacological effects. Examples of alkaloids according to the invention are N-(2-methylaminobenzoyl) tryptamine, Dihydrocapsaicin, or Leonurine.

Benzophenone derivatives refer to a class of organic compounds that are structurally derived from benzophenone, which is a ketone (a carbonyl compound) consisting of two benzene rings connected by a carbonyl group (C6H5C(O)C6H5). Benzophenone derivatives are obtained by modifying or substituting functional groups on the benzophenone structure. Example of Benzophenone derivative according to the invention is Iriflophenone 3-c-beta glucoside.

Diarylheptanoids are a class of organic compounds characterized by a structural motif consisting of two aryl (phenyl) rings connected by a heptane chain. These compounds belong to the larger group of polyphenolic natural products and are commonly found in certain plants, particularly in the Zingiberaceae family, which includes ginger and turmeric. The basic structure of diarylheptanoids consists of two aromatic rings (aryl groups) linked by a seven-carbon aliphatic chain (heptane). Example of Diarylheptanoids according to the invention is 1-(3,4-dihydroxyphenyl)-7-(4-hydroxyphenyl)-3,5-heptanediol.

Diterpenoids are a class of organic compounds that belong to the terpenoid family, specifically the diterpene subclass. Terpenoids are natural products derived from isoprene units (C5H8), and diterpenoids consist of four isoprene units, resulting in a 20-carbon structure. These compounds are widespread in nature and can be found in various plants, fungi, and some marine organisms. The basic structural unit of a diterpenoid is a C20 skeleton, often arranged in the linear form or in cyclized form (up to four fused rings) of four linked isoprene units (C5H8). Examples of Diterpenoids according to the invention is Uncinatone.

Flavonoids are a class of polyphenolic compounds widely distributed in the plant kingdom. They are characterized by a 2-phenylchromane core skeleton composed of 15-carbon skeleton containing two phenyl rings (A and B rings) and a heterocyclic ring (C ring) with oxygen. The specific arrangement and substitution of these rings lead to the diversity within the flavonoid class. Flavonoids can be further categorized into subgroups such flavones, flavonols, flavanones, isoflavones, flavans, flavanols, chalcones, anthocyanins, and isoflavonoids or neoflavonoids, each with unique structural variations. Examples of Flavonoids according to the invention are Kuwanon E or Angeflorin.

Homoisoflavonoids are a class of organic compounds that belong to the larger family of flavonoids. They share structural similarities with conventional isoflavonoids but are distinguished by an additional carbon between the B and C rings on the isoflavonoid skeleton (general structure of a 16-carbon skeleton. Example of Homoisoflavonoids according to the invention is (r)-3-(3,4-dihydroxybenzyl)-3,7-dihydroxy-4-chromanone (also named Sappanone B).

Lignans are a class of polyphenolic compounds found in plants, particularly in seeds, grains, vegetables, and fruits. They are characterized by their chemical structure, which typically consists of two phenylpropane units linked together. The basic unit of lignans is a dibenzylbutane skeleton. Examples of Lignans according to the invention are Mesosecoisolariciresinol, Arctiin or Silandrin

Phenolic compounds, also known as phenols, are a diverse group of chemical compounds characterized by the presence of a phenol group, which consists of a hydroxyl (-OH) group attached to an aromatic benzene ring. These compounds can be found in various natural sources, including plants, fruits, vegetables, and beverages such as tea and wine. Examples of Phenolic compounds according to the invention are cristatic acid, Itoside a, Parishin c, or 2-(3,4-dihydroxyphenyl)ethyl beta-d-glucopyranoside.

Phenylpropanoids are a class of organic compounds derived from the aromatic amino acid phenylalanine. They play a significant role in plant metabolism and are widely distributed in the plant kingdom. The structural backbone of phenylpropanoids consists of a three-carbon propene chain (propenyl) linked to a phenyl ring. Examples of Phenylpropanoids compounds according to the invention are 3-(3-hydroxy-1-oxo-2-phenylpropoxy)-2-phenylpropanoic acid, Calceolarioside B, 9'''-methyl salvianolate b, or Icariside h1.

Phloracetophenone glucoside is a glycoside of Phloracetophenone, both natural products found in different plants. Phloracetophenone is a benzenetriol derived from acetophenone in which the hydrogens at positions 2, 4, and 6 on the phenyl group are replaced by hydroxy groups. The structure of phloracetophenone combines features of both ketones and phenols : it is a methyl ketone, a benzenetriol and an aromatic ketone. Example of Phloracetophenone glucoside according to the invention is Phloracetophenone 4'-o-glucoside

Polyketides are a class of natural products that are biosynthesized by enzymatic condensation of building blocks formed by a starter unit (typically acetyl-CoA or propionyl-CoA) with an extender unit (either malonyl-CoA or methylmalonyl-CoA). These building blocks form a chain characterized by a repeating series of ketone groups along their carbon backbone. Polyketides then undergo functional and structural modifications to reach their final form. Example of Polyketide according to the invention is Annullatin c.

Stilbenoids are a class of natural compounds that belong to the larger group of phytochemicals. Stilbenoids are characterized by the presence of a stilbene backbone in their chemical structure. The basic structure of a stilbenoid is a 1 ,2-diphenylethylene (stilbene) molecule, which consists of two phenyl (aromatic) rings linked by a double bond (-CH=CH-). Stilbenoids may have various substituents and functional groups attached to this basic structure, leading to a diverse array of compounds within this class. Example of Stilbenoids according to the invention is Salvianolic acid a

Related to all aspects of the invention, in an embodiment, said at least one compound in a cosmetically acceptable medium is at least one compound among Calceolarioside B, Arctiin, Meso-secoisolariciresinol, Kuwanon E, and/or Iriflophenone 3-c-beta-glucoside.

Preferably, these at least one compounds are in a cosmetically effective amount in the compositions according to the invention.

In an embodiment, said composition comprise Kuwanon E, in a cosmetically acceptable medium, as active ingredient to inhibit the activity of KLF4.

In an aspect, the invention concerns a cosmetic non-therapeutic composition comprising Kuwanon E, in a cosmetically acceptable medium, as active ingredient to inhibit the activity of KLF4 to inhibit the activity of Kruppel-like factor 4 (KLF4).

In another aspect, the invention concerns a non-therapeutic cosmetic process for skin and/or scalp and/or hair and/or mucous membranes anti-aging care, comprising the topical application of a composition comprising Kuwanon E, in a cosmetically acceptable medium.

In another aspect, the invention concerns the use of a cosmetic non-therapeutic composition comprising Kuwanon E, in a cosmetically acceptable medium, to inhibit the activity of KLF4.

In an embodiment, said composition comprise Calceolarioside B, in a cosmetically acceptable medium, as active ingredient to inhibit the activity of KLF4.

In an aspect, the invention concerns a cosmetic non-therapeutic composition comprising Calceolarioside B, in a cosmetically acceptable medium, as active ingredient to inhibit the activity of KLF4 to inhibit the activity of Kruppel-like factor 4 (KLF4).

In another aspect, the invention concerns a non-therapeutic cosmetic process for skin and/or scalp and/or hair and/or mucous membranes anti-aging care, comprising the topical application of a composition comprising Calceolarioside B, in a cosmetically acceptable medium.

In another aspect, the invention concerns the use of a cosmetic non-therapeutic composition comprising Calceolarioside B, in a cosmetically acceptable medium, to inhibit the activity of KLF4.

In an embodiment, said composition comprise Arctiin, in a cosmetically acceptable medium, as active ingredient to inhibit the activity of KLF4.

In an aspect, the invention concerns a cosmetic non-therapeutic composition comprising Arctiin, in a cosmetically acceptable medium, as active ingredient to inhibit the activity of KLF4 to inhibit the activity of Kruppel-like factor 4 (KLF4).

In another aspect, the invention concerns a non-therapeutic cosmetic process for skin and/or scalp and/or hair and/or mucous membranes anti-aging care, comprising the topical application of a composition comprising Arctiin, in a cosmetically acceptable medium.

In another aspect, the invention concerns the use of a cosmetic non-therapeutic composition comprising Arctiin, in a cosmetically acceptable medium, to inhibit the activity of KLF4.

In an embodiment, said composition comprise Iriflophenone 3-c-beta-glucoside, in a cosmetically acceptable medium, as active ingredient to inhibit the activity of KLF4.

In an aspect, the invention concerns a cosmetic non-therapeutic composition comprising Iriflophenone 3-c-beta-glucoside, in a cosmetically acceptable medium, as active ingredient to inhibit the activity of KLF4 to inhibit the activity of Kruppel-like factor 4 (KLF4).

in another aspect, the invention concerns a non-therapeutic cosmetic process for skin and/or scalp and/or hair and/or mucous membranes anti-aging care, comprising the topical application of a composition comprising Iriflophenone 3-c-beta-glucoside, in a cosmetically acceptable medium.

In another aspect, the invention concerns the use of a cosmetic non-therapeutic composition comprising Iriflophenone 3-c-beta-glucoside, in a cosmetically acceptable medium, to inhibit the activity of KLF4.

In an embodiment, said composition comprise Meso-secoisolariciresinol, in a cosmetically acceptable medium, as active ingredient to inhibit the activity of KLF4.

In an aspect, the invention concerns a cosmetic non-therapeutic composition comprising Meso-secoisolariciresinol, in a cosmetically acceptable medium, as active ingredient to inhibit the activity of KLF4 to inhibit the activity of Kruppel-like factor 4 (KLF4).

In another aspect, the invention concerns a non-therapeutic cosmetic process for skin and/or scalp and/or hair and/or mucous membranes anti-aging care, comprising the topical application of a composition comprising Meso-secoisolariciresinol, in a cosmetically acceptable medium.

In another aspect, the invention concerns the use of a cosmetic non-therapeutic composition comprising Meso-secoisolariciresinol, in a cosmetically acceptable medium, to inhibit the activity of KLF4.

Kuwanon E is a flavonoid isolated found in the root bark of Morus alba. The chemical structure of this molecule is:

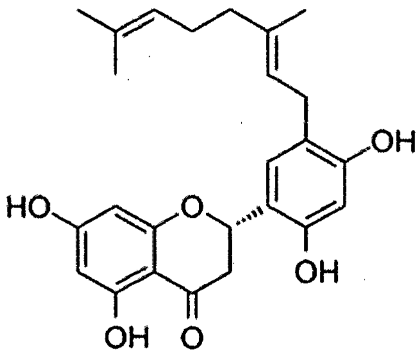

Calceolarioside B is a hydroxycinnamic acid of chemical formula:

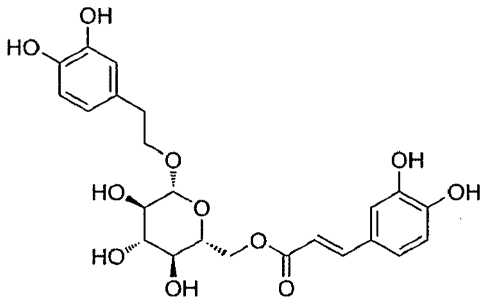

Arctiin is a lignan found in many plants of the family Asteraceae, of chemical formula:

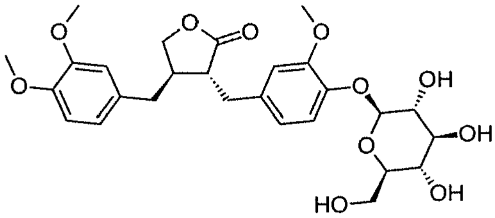

Iriflophenone 3-c-beta-glucoside is a compound of Mangifera indica leaves extract, of chemical formula:

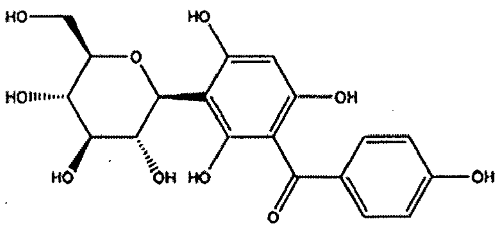

Meso-secoisolariciresinol is an enterolignan precursor, of chemical formula:

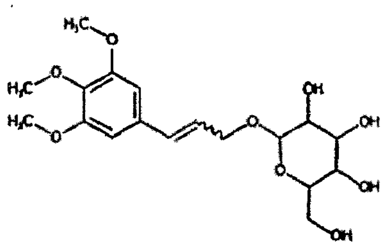

Preferably, the concentration of said at least one of Calceolarioside B, Arctiin, Meso-secoisolariciresinol, Kuwanon E, and/or Iriflophenone 3-c-beta-glucoside in the composition is from 0.0001% to 10% by weight of the total composition.

Preferably, said concentration of said at least one of Calceolarioside B, Arctiin, Meso-secoisolariciresinol, Kuwanon E, and/or Iriflophenone 3-c-beta-glucoside in the composition is from 0.0001% to 5% by weight of the total composition.

In an embodiment, said concentration of said at least one of Calceolarioside B, Arctiin, Meso-secoisolariciresinol, Kuwanon E, and/or Iriflophenone 3-c-beta-glucoside in the composition is chosen among, by percentage of weight of the total composition: 0.0001, 0.0005, 0.001, or 0,005, or 0,01, or 0,05, or 0,10, or 0,15, or 0,20, or 0,25, or 0,30, or 0,35, or 0,40, or 0,45, or 0,50, or 0,55, or 0,60, or 0,65, or 0,70, or 0,75, or 0,80, or 0,85, or 0,90, or 0,95, or 1, or 1,25, or 1,50, or 1,75, or 2, or 2,25, or 2,50, or 2,75, or 3, or 3,25, or 3,50, or 3,75, or 4, or 4,25, or 4,50, or 4,75, or 5, or 5,25, or 5,50, or 5,75, or 6, or 6,25, or 6,50, or 6,75, or 7, or 7,25, or 7,50, or 8, or 8,25, or 8,50, or 8,75, or 9, or 9,25, or 9,50, or 9,75, or 10.

Preferably, Kuwanon E is included in the composition through Morus alba extract, more preferably from the root bark.

Preferably, Calceolarioside B is included in the composition through the addition of at least one extract chosen among Plantago asiatica extract, Plantago major extract, Plantago lanceolata extract, Cirsium setosum extract, Houttuynia cordata extract, or Forsythia suspensa extract.

Preferably, Arctiin is included in the composition through the addition of at least one extract chosen among Arctium lappa extract, Forsythia suspensa extract, Daphne feddei extract, Torreya nucifera extract, Saussurea involucrata extract, Eucommia ulmoides extract, Lycium barbarum extract.

Preferably, Iriflophenone 3-c-beta-glucoside is included in the composition through the addition of at least one extract chosen among Mangifera indica extract or Aquilaria sinensis extract, more preferably from the leaves.

Preferably, Meso-secoisolariciresinol is included in the composition through the addition of at least one extract chosen among Citrullus lanatus extract, Cedrus deodara extract, Arctium lappa extract, Juniperus chinensis extract, Annona squamosa extract, Sedum sarmentosum extract, Santalum album extract, Sargentodoxa cuneata extract, Daphne feddei extract, or Larix sibirica extract, or Osmanthus fragrans extract.

The composition according to the invention is suitable for topical application to the skin, and/or scalp and/or hair and/or mucous membranes and therefore generally comprises a cosmetically acceptable medium, that is to say a medium compatible with the skin and/or scalp and/or hair and/or mucous membranes" and which has a pleasant color, an odor and a pleasant feel and which does not generate unacceptable discomfort (stinging, tautness, redness), capable of diverting the consumer from using this composition.

In a known manner, the composition of the invention may also contain adjuvants which are common in the cosmetic field, such as hydrophilic or lipophilic gelling agents, hydrophilic or lipophilic active agents, moisturizing agents (such as glycerin, propylene glycol, butylene glycol, pentylene glycol, hexylene glycol, dipropylene glycol, diethylene glycol), preservatives, antioxidants, solvents, perfumes, fillers, pigments, hydrophilic screening agents, odor absorbers and dyestuffs. The amounts of these various adjuvants are those conventionally used in the fields under consideration, and for example from 0,01 to 20% of the total weight of the composition. These adjuvants, depending on their nature, can be introduced into the fatty phase, into the aqueous phase, into the lipid vesicles and/or into the nanoparticles.

A person skilled in the art will take care to select the optional compound(s) to be added to the compositions according to the invention, as well as their concentration, such that the advantageous properties intrinsically associated with the compositions in accordance with the invention are not, or are not substantially, adversely affected by the envisaged addition.

The composition according to the invention may be presented in any galenic form normally used for cosmetic topical application to the skin and/or scalp and/or hair and/or mucous membranes.

Related to all aspects of the invention, said composition preferably is formulated in the form of a cream, an ointment, a mask, a serum, a milk, a lotion, a paste, a foam, an aerosol, a stick, a shampoo, a conditioner, patches, an aqueous-alcoholic or oily solution, an oil-in-water or water-in-oil or multiple emulsion, an aqueous or oily gel, a liquid, pasty or solid anhydrous product, a dispersion of oil in an aqueous phase using spherules, these spherules being polymeric nanoparticles such as nanospheres and nanocapsules or lipid vesicles of ionic and/or non-ionic type.

Preferably, the cosmetic composition according to the invention is applied once or twice a day, preferably 2 times a day, for at least 7 days, preferably at least 15 days, even more preferably at least one month, particularly preferably at least 2 months, and most preferably at least 3 months. Particularly preferably, the cosmetic composition is applied in the process according to the invention 2 times a day for at least 3 months.

In other embodiments according to the invention, the composition according to the invention may comprise in a cosmetically acceptable medium at least one of : Annullatin c, Phloracetophenone 4'-o-glucoside, Adlerol, Cristatic acid, 9'''-methyl salvianolate b, Parishin c, N-(2-methylaminobenzoyl)tryptamine, Salvianolic acid a, Dihydrocapsaicin, Silandrin, Angeflorin, 2-(3,4-dihydroxyphenyl)ethyl beta-d-glucopyranoside, 3-(3-hydroxy-1-oxo-2-phenylpropoxy)-2-phenylpropanoic acid, 1-(3,4-dihydroxyphenyl)-7-(4-hydroxyphenyl)-3,5-heptanediol, Itoside a, Leonurine, Uncinatone,

(r)-3-(3,4-dihydroxybenzyl)-3,7-dihydroxy-4-chromanone, and/or Icariside h1.

In an aspect, the invention concerns a cosmetic non-therapeutic composition comprising Annullatin c, Phloracetophenone 4'-o-glucoside, Adlerol, Cristatic acid, 9'''-methyl salvianolate b, Parishin c, N-(2-methylaminobenzoyl) tryptamine, Salvianolic acid a, Dihydrocapsaicin, Silandrin, Angeflorin, 2-(3,4-dihydroxyphenyl)ethyl beta-d-glucopyranoside, 3-(3-hydroxy-1-oxo-2-phenylpropoxy)-2-phenylpropanoic acid, 1-(3,4-dihydroxyphenyl)-7-(4-hydroxyphenyl)-3,5-heptanediol, Itoside a, Leonurine, Uncinatone, (r)-3-(3,4-dihydroxybenzyl)-3,7-dihydroxy-4-chromanone, and/or Icariside h1, in a cosmetically acceptable medium, as active ingredient to inhibit the activity of KLF4 to inhibit the activity of Kruppel-like factor 4 (KLF4).

In another aspect, the invention concerns a non-therapeutic cosmetic process for skin and/or scalp and/or hair and/or mucous membranes anti-aging care, comprising the topical application of a composition comprising Annullatin c, Phloracetophenone 4'-o-glucoside, Adlerol, Cristatic acid, 9'''-methyl salvianolate b, Parishin c, N-(2-methylaminobenzoyl)tryptamine, Salvianolic acid a, Dihydrocapsaicin, Silandrin, Angeflorin, 2-(3,4-dihydroxyphenyl)ethyl beta-d-glucopyranoside, 3-(3-hydroxy-1-oxo-2-phenylpropoxy)-2-phenylpropanoic acid, 1-(3,4-dihydroxyphenyl)-7-(4-hydroxyphenyl)-3,5-heptanediol, Itoside a, Leonurine, Uncinatone, (r)-3-(3,4-dihydroxybenzyl)-3,7-dihydroxy-4-chromanone, and/or Icariside h1, in a cosmetically acceptable medium.

In another aspect, the invention concerns the use of a cosmetic non-therapeutic composition comprising Annullatin c, Phloracetophenone 4'-o-glucoside, Adlerol, Cristatic acid, 9'''-methyl salvianolate b, Parishin c, N-(2-methylaminobenzoyl)tryptamine, Salvianolic acid a, Dihydrocapsaicin, Silandrin, Angeflorin, 2-(3,4-dihydroxyphenyl) ethyl beta-d-glucopyranoside, 3-(3-hydroxy-1-oxo-2-phenylpropoxy)-2-phenylpropanoic acid, 1-(3,4-dihydroxyphenyl)-7-(4-hydroxyphenyl)-3,5-heptanediol, Itoside a, Leonurine, Uncinatone, (r)-3-(3,4-dihydroxybenzyl)-3,7-dihydroxy-4-chromanone, and/or Icariside h1, in a cosmetically acceptable medium, to inhibit the activity of KLF4.

The concentration of said at least one of Annullatin c, Phloracetophenone 4'-o-glucoside, Adlerol, Cristatic acid, 9'''-methyl salvianolate b, Parishin c, N-(2-methylaminobenzoyl)tryptamine, Salvianolic acid a, Dihydrocapsaicin, Silandrin, Angeflorin, 2-(3,4-dihydroxyphenyl)ethyl beta-d-glucopyranoside, 3-(3-hydroxy-1-oxo-2-phenylpropoxy)-2-phenylpropanoic acid, 1-(3,4-dihydroxyphenyl)-7-(4-hydroxyphenyl)-3,5-heptanediol, Itoside a, Leonurine, Uncinatone, (r)-3-(3,4-dihydroxybenzyl)-3,7-dihydroxy-4-chromanone, and/or Icariside h1, in the composition is from 0.0001% to 10% by weight of the total composition.

The concentration of said at least one of Annullatin c, Phloracetophenone 4'-o-glucoside, Adlerol, Cristatic acid, 9'''-methyl salvianolate b, Parishin c, N-(2-methylaminobenzoyl)tryptamine, Salvianolic acid a, Dihydrocapsaicin, Silandrin, Angeflorin, 2-(3,4-dihydroxyphenyl)ethyl beta-d-glucopyranoside, 3-(3-hydroxy-1-oxo-2-phenylpropoxy)-2-phenylpropanoic acid, 1-(3,4-dihydroxyphenyl)-7-(4-hydroxyphenyl)-3,5-heptanediol, Itoside a, Leonurine, Uncinatone, (r)-3-(3,4-dihydroxybenzyl)-3,7-dihydroxy-4-chromanone, and/or Icariside h1, in the composition is from 0.1% to 5% by weight of the total composition.

In an embodiment, said concentration of said at least one of Annullatin c, Phloracetophenone 4'-o-glucoside, Adlerol, Cristatic acid, 9'''-methyl salvianolate b, Parishin c, N-(2-methylaminobenzoyl)tryptamine, Salvianolic acid a, Dihydrocapsaicin, Silandrin, Angeflorin, 2-(3,4-dihydroxyphenyl)ethyl beta-d-glucopyranoside, 3-(3-hydroxy-1-oxo-2-phenylpropoxy)-2-phenylpropanoic acid, 1-(3,4-dihydroxyphenyl)-7-(4-hydroxyphenyl)-3,5-heptanediol, Itoside a, Leonurine, Uncinatone, (r)-3-(3,4-dihydroxybenzyl)-3,7-dihydroxy-4-chromanone, and/or Icariside h1, in the composition is chosen among, by percentage of weight of the total composition: 0.0001, 0.0005, 0.001, or 0,005, or 0,01, or 0,05, or 0,10, or 0,15, or 0,20, of 0,25, or 0,30, or 0,35, or 0,40, or 0,45, or 0,50, or 0,55, or 0,60, or 0,65, or 0,70, or 0,75, or 0,80, or 0,85, or 0,90, or 0,95, or 1, or 1,25, or 1,50, or 1,75, or 2, or 2,25, or 2,50, or 2,75, or 3, or 3,25, or 3,50, or 3,75, or 4, or 4,25, or 4,50, or 4,75, or 5, or 5,25, or 5,50, or 5,75, or 6, or 6,25, or 6,50, or 6,75, or 7, or 7,25, or 7,50, or 8, or 8,25, or 8,50, or 8,75, or 9, or 9,25, or 9,50, or 9,75, or 10.

Chemical formula of these compounds:

| Molecule | Chemical formula |
|---|---|
| Adlerol | 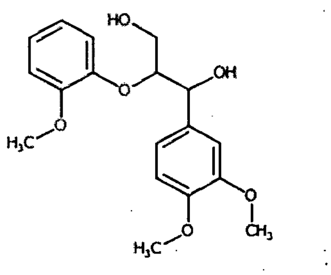 |

(continued)

| Molecule | Chemical formula |
|---|---|
| Cristatic acid | 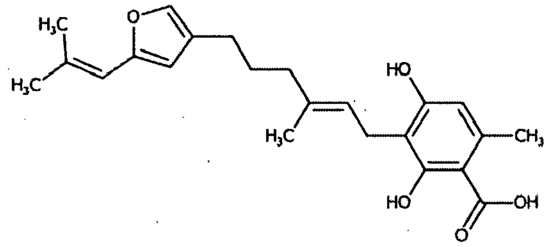 |
| Parishin c | 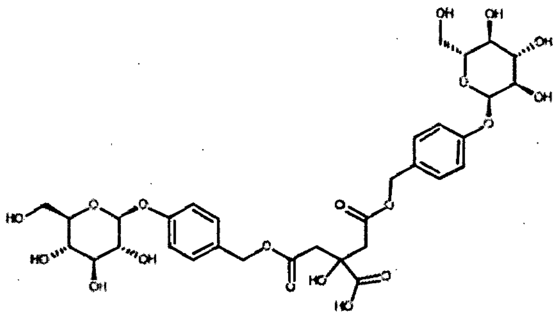 |
| Salvianolic acid a | 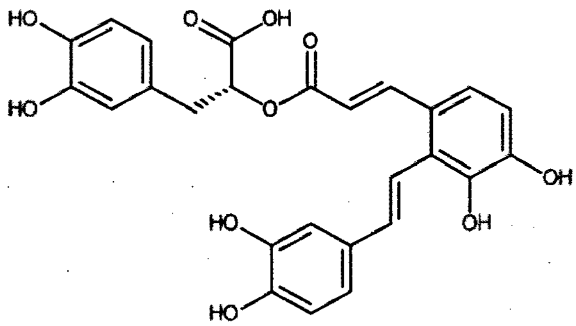 |
| Silandrin | 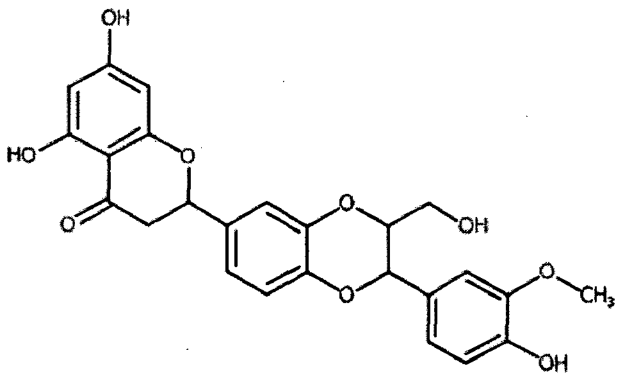 |
| 2-(3,4- dihydroxyphen yl)ethyl beta-d- glucopyranoside | 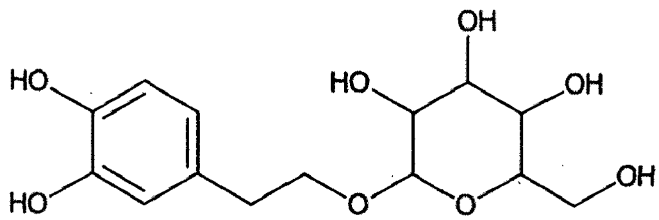 |

(continued)

| Molecule | Chemical formula |
|---|---|
| 1-(3,4- dihydroxyphen yl)-7-(4-hydroxyphenyl) -3,5- heptanediol | 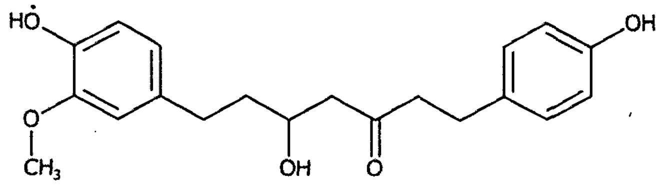 |
| Itoside a | 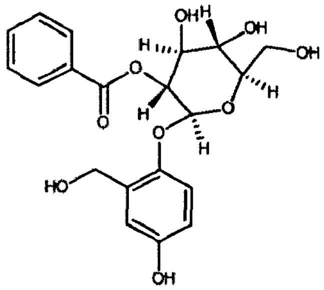 |
| Leonurine | 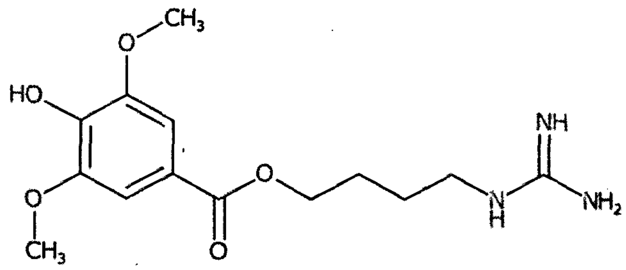 |
| (r)-3-(3,4- dihydroxybenz yl)-3,7-dihydroxy-4- chromanone | 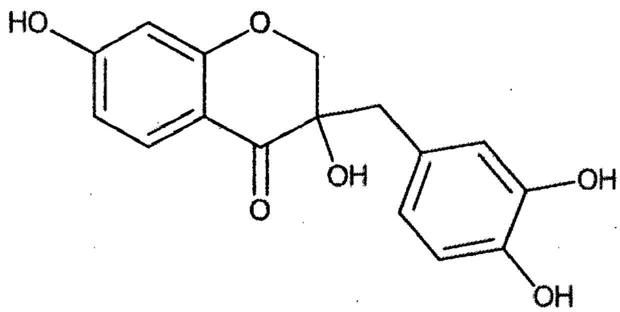 |
| Annullatin c | 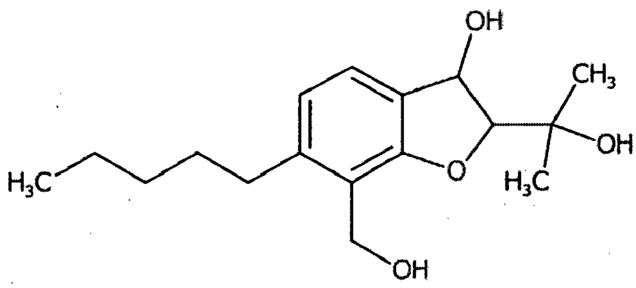 |
| Phloracetophe none 4'-o- glucoside | 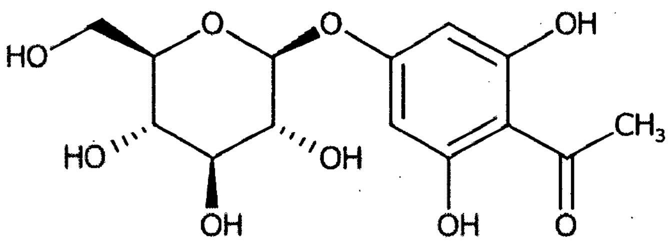 |

(continued)

| Molecule | Chemical formula |
|---|---|
| 9'''-methyl salvianolate b | 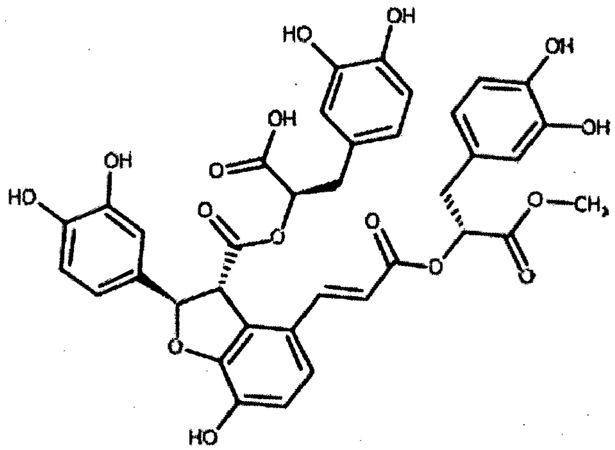 |
| N-(2-methylaminob enzoyl)trypta-mi ne | 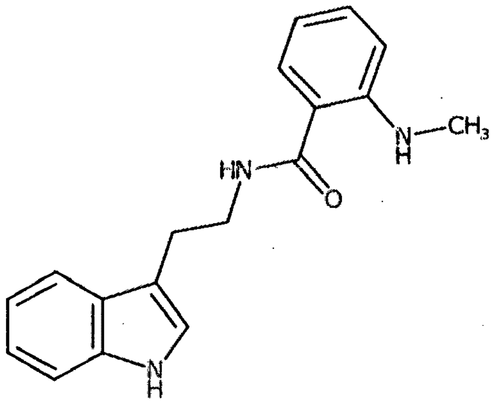 |
| Dihydrocapsaicin | 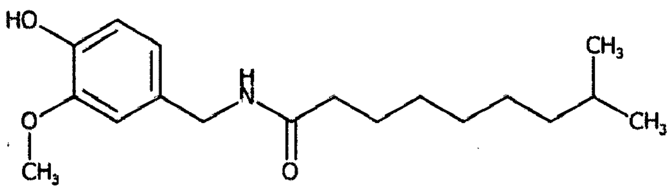 |
| Angeflorin | 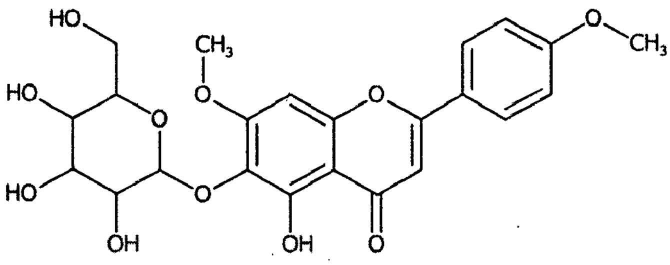 |

(continued)

| Molecule | Chemical formula |
|---|---|
| 3-(3-hydroxy-1-oxo-2-phenylpropoxy)-2-phenylpropano ic acid | 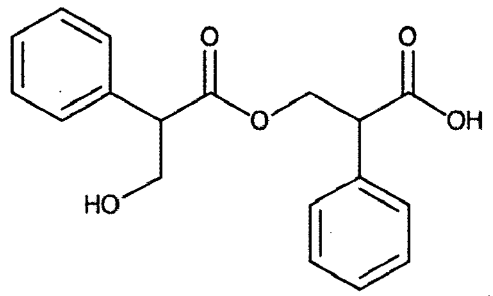 |
| Uncinatone | 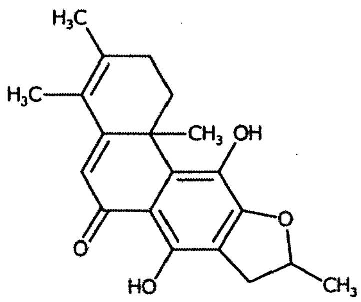 |
| Icariside h1 | 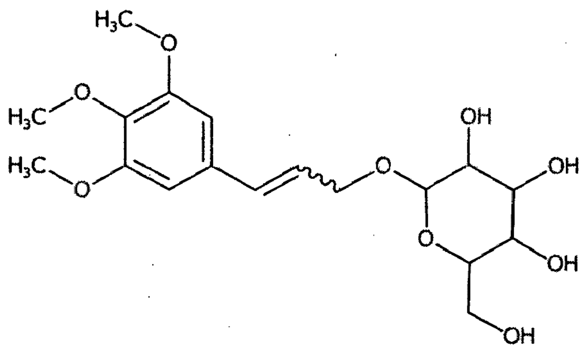 |

These compounds may be comprised in said composition according to the invention, in replacement or in addition of Calceolarioside B, Arctiin, Meso-secoisolariciresinol, Kuwanon E, and/or Iriflophenone 3-c-beta-glucoside.

Kuwanon E, Silandrin, Leonurine, (r)-3-(3,4- dihydroxybenz yl)-3,7- dihydroxy-4- chromanone, Annullatin c, Phloracetophe none 4'-o- glucoside, Dihydrocapsaicin, Iriflophenone 3-c-beta-glucoside, Meso-secoisolaricires inol, and Icariside h1 are bonding to PRMT5.

Parishin c, Arctiin, 2-(3,4- dihydroxyphen yl)ethyl beta-d- glucopyranoside, 9'''-methyl salvianolate b, N-(2-methylaminobenzoyl)tryptamine, 3-(3-hydroxy-1- oxo-2- phenylpropoxy )-2-phenylpropano ic acid, Uncinatone are bonding to KLF4.

Calceolarioside B, Adlerol, Cristatic acid, Salvianolic acid a, 1-(3,4- dihydroxyphen yl)-7-(4-hydroxyphenyl) -3,5-heptanediol, Itoside a, Angeflorin, are inhibiting the interaction between KLF4 and PRMT5.

For the purposes of the invention, "non-therapeutic" means a cosmetic application that is not intended to treat a patient. In fact, the cosmetic use according to the invention does not act as a drug for the treatment of diseases, but only to attenuate the visual signs of ageing on the skin and/or scalp, and/or hair and/or mucous membranes.

In an embodiment, the user is a mammal, including non-human mammal, and is, in particular, a human being. In a preferred embodiment, the user is a human adult.

By "topical application" is meant in the sense of the present invention an application to the skin, including the scalp, and hair, and the mucous membranes.

In an embodiment, said cosmetic composition also comprises at least one other cosmetically acceptable agent, preferably chosen from soothing agents, restructuring agents, regenerating agents, revitalizing agents, sunscreens, anti-wrinkle agents, moisturizing agents, anti-aging agents, surfactants, fats, organic solvents, solubilizing agents, thickening and gelling agents, smoothing agents, firmness-enhancing agents, elasticity and/or the barrier effect of the skin, antioxidants, opacifiers, thermal waters, mattifying agents, chemical or mineral filters, trace elements, stabilizing agents,

foaming agents, perfumes, ionic or non-ionic emulsifiers, fillers, sequestrants and chelators, perfumes, filters, essential oils, coloring matters, pigments, hydrophilic or lipophilic active ingredients, lipid vesicles encapsulating one or more active ingredients and/or preservatives.

By "cosmetically acceptable" is meant according to the present invention a compound which is useful in the preparation of a cosmetic composition, is generally safe, non-toxic and neither biologically nor otherwise undesirable, and is acceptable for cosmetic use, particularly by topical application to the skin including the scalp, and hair, and the mucous membranes.

By "cosmetically" or effective amount is meant an amount that achieves the effects claimed according to the invention.

In an embodiment, an amount of 0.2 to 3 mg/cm2 of the cosmetic composition is applied to the skin, scalp, hair or mucous membranes area, preferably 2 mg/cm2.

In the description and in the following examples, unless otherwise indicated, percentages are percentages by weight and ranges of values expressed as "between ... and ..." include the upper and lower limits specified. The following examples are illustrative and non-limiting of the scope of the invention.

## Brief Description of Drawings

[Fig.1]: Stiffness of the dermal matrix with aging. (A) Atomic force microscopy (AFM) study of human skin. Left, sagittal tissue sections (10 μm) were prepared from full thickness skin biopsies and used for AFM measurements. The AFM cantilever with a spherical probe tip of 140 nm diameter was navigated in the dermal matrix on the top (T) and bottom (B) of the epidermal rete ridges using light microscopy, bar 20 μm. Right, for all samples, 16 × 16 points were examined in a 5 × 5 μm area as shown in the colour-coded maps, scale bar 300 nm. (B) Force-displacement curves (F-z) were generated for each point by plotting the deflection of the cantilever against the controlled deformation. E was calculated by fitting the contact part of the force-displacement curves (F-d) using a standard Hertz model. (C) AFM assessment of human skin. Elastic modulus (E) values of the dermal ECM at the top and bottom of rete ridges of abdominal skin biopsies from young and old donors. Each scatter plot represents the E of the force curve from 1 of the 256 spots in 10 rete ridges of each skin biopsy specimen from six independent donors, including three younger donors at 20, 20, and 26 years of age, and three elderly donors at 51, 51, and 59 years of age. Shown are means ± SD with ****p < 0.0001; ANOVA.

[Fig.2]: (A) C- DIC phase contrast images of two strains of primary human keratinocytes (strain 1: foreskin, strain 2: abdomen at age 32) cultured for 48 h on surfaces with stiffness of 2 KPa and 32 KPa, as indicated. Bar, 50 μm. (B) Expression of COL17A1, LAMA3, PLEC, and DST mRNA in primary foreskin (strain 1) and abdominal skin (strain 2; from a 36-year-old donor) keratinocytes cultured for 48 h on supports with stiffness of 2 and 32 kPa, as indicated. The graph summarizes data from three independent experiments. Each symbol represents the gene/GAPDH ratio determined for a single well in each experiment. Means ± SD are presented, with significance at **p < 0.01 as determined by Student's t-tests.

[Fig.3]: Histograms generated from the mRNA expression data reflect the differential expression of six genes in the rete-ridges of foreskin (FS) compared to the abdominal skin of young (Y) and old (O) donors determined by NanoString nCounter® analysis. Each symbol corresponds to a single donor sample.

[Fig.4]: (A) Immunofluorescence staining of KLF4 on human frozen sections from human biopsies of the indicated origin. KLF4 antibody was detected with CY3-labelled antibody and nuclei were stained with DAPI. Images of CY3-labelled staining and DAPI staining are combined. Bar, 40 μm. (B) Determination of the KLF4/DAPI immunostaining ratio in the epidermis of human foreskin and abdominal skin samples, the latter divided into two age groups as shown. Each group includes skin samples from at least three different donors. Each independent experiment was repeated twice. Means ± SD are presented with ****p < 0.0001 determined by ANOVA.

[Fig.5]: (A) KLF4 mRNA expression obtained by RT-PCR from the two strains of primary human keratinocytes (strain 1: foreskin, strain 2: abdomen at age 32) cultured for 48 h on surfaces with stiffness of 2 KPa and 32 KPa. (B) Expression of KLF4 mRNA in primary keratinocytes isolated from strains 1 and 2 as indicated in (c) and cultured for 48 h on supports with stiffness of 2 and 32 kPa. The graph summarizes the data from three independent experiments for each of the strains evaluated. Each symbol represents the gene/GAPDH ratio determined for a single well in each experiment. Means ± SD are presented with ***p < 0.001 determined by Student's t-tests.

[Fig.6]: Expression of KLF4 in normal human keratinocytes in culture. A. Detection of KLF4 protein in keratinocytes isolated in culture from biopsies of the foreskin (FS) or abdomen of donors aged 36 and 44 years using an in *cellulo* Elisa assay. Each symbol represents a single experiment. Shown are means ± SD with ****p < 0.0001, determined by an ANOVA test. B. Expression of *KLF4* and *GAPDH* mRNA in cultured primary keratinocytes isolated from the strains described in (A). C. Modulation of KLF4 protein expression in the three strains of human keratinocytes described in (A) in culture by 40 ng/ml TGFα and 1.2 mM Cacl2. D. Modulation of KLF4 protein expression in the strains of human keratinocytes from donors aged 36 (solid black symbol) and 44 (empty black symbol) in culture by the indicated concentrations of Kuwanon E, Calceolarioside B, Arctiin, and Meso-secoisolariciresinol. Controls with 40 ng/ml TGFα and 1.2 mM Cacl2 are shown. (A, C, D) Each symbol represents a single well experiment. Shown are means ± SD with ****p < 0.0001, **p < 0.01, *p < 0.1 determined by an ANOVA test.

## Examples

EXAMPLE 1: Molecular and spatial changes of the epidermal basement membrane identified in aging skin:

Because the characteristic configuration of the DEJ may serve as critical support for epidermal homeostasis, the goal is to determine the contributions of its molecular composition to the formation and maintenance of rete ridges. This study analyzes the expression of DEJ components along the rete ridges in human skin, tracked their fate, and performed experiments to determine the impact of tissue stiffness on their changes associated with aging.

MATERIALS AND METHODS

Collection of skin specimens

Foreskin samples were collected from eight male infants (mean age, 4 years old) who were undergoing circumcision. Biopsies of UV-unexposed skin were collected from female patients who had undergone breast or abdominal reduction following ethical and safety guidelines according to French regulations. Verbal and written, informed consent was obtained from all participants who provided skin samples according to declaration no. DC-2008-162 that was submitted to the Cell and Tissue Bank of the Hospices Civils de Lyon. The 12 female participants enrolled in this part of the study included five elderly donors (aged 51-71 years), four middle-aged donors (ages 35-47 years), and five young donors (aged 20-32 years). Surgical specimens were embedded in Tissue-Tek OCT (Microm Microtech, Brignais, France).

Atomic force microscopy

AFM indentation experiments were performed in PBS at RT using a Bioscope II Catalyst (Bruker Nanosurfaces, Santa Barbara, CA, USA; driven by Nanoscope v. 9.13) mounted on a DMI600 inverted optical microscope (Leica, driven by Micro-Manager 2.0-gamma) that was equipped with a 20x dry objective. Pre-calibrated PFQNM-LC-A-CAL cantilevers (Bruker) were used with a nominal spring constant of 0.1 N/m and a nominal tip radius of 70 nm. Deflection sensitivity was calibrated by generating a force curve on a sapphire disc in PBS. The FV mode was used to measure Young's modulus (E). In this mode, a series of force curves were generated along a matrix defined for the ROI of the sample. For these experiments, 16 × 16 force curves were acquired for 5 × 5 $\mu m^2$ areas, which yielded 256 curves for each zone (Figure 1B). For all measurements, the curve parameters included a ramp size of 2 μm, a ramp rate of 1 Hz, and a trigger force of 1 nN, corresponding on average to an indentation of 300 nm; this will be small enough compared to the distance between indentation positions so that they will not be influenced by one another. A minimum of 10 rete ridges were evaluated in each biopsy specimen, including the ECM at the top and bottom of each ridge. Hydrated sagittal cryosections (10-μm) were examined and the E of the dermis and epidermis were measured. The glass slide was positioned and blocked on a motorized XY stage and 100 μl of PBS was added to the sample. The tip was driven to the ROI using an optical microscope. Each curve was analyzed using the Nanoscope Analysis software (Bruker Nanosurfaces, v. 2.0) to determine the average E. A baseline correction algorithm was used to remove any tilt and set the average to 0 force. We fit each force-versus-indentation curve using the classical Hertz model:

$$F(\delta) = \frac{4}{3}\frac{E}{1-\nu^2} \cdot \sqrt{R} \cdot \delta^{3/2}$$

where E is the relative apparent Young's modulus, v is the Poisson ratio of the sample (i.e., 0.5 for our samples that were incompressible, as generally assumed for biological material and specifically confirmed for skin sample (Li et al., 2012), R is the nominal radius of the tip and δ is the indentation. All the fits were performed between 10% and 90% of the trigger force.

R is the nominal radius of the tip, and $\delta$ is the indentation. All curve fitting was performed using 10%-90% of the trigger force. In its strictest sense, the Hertz model is only applicable to elastic, homogeneous, and isotropic materials. However, E is generally accepted as a measure of the mechanical properties of tissues responding to small strains, which is the case for AFM-generated indentations.

Microdissection, RNA extraction and transcriptomic analysis

Twelve human skin samples were fixed in formalin and embedded in paraffin. Tissue sections (10 $\mu$m thick) were cut for laser capture microdissection. The tissue samples were mounted on PEN Membrane Glass Slides (Applied Biosystems) and dried overnight at RT. To visualize the areas of interest, the tissue sections were deparaffinized in two baths of xylene and one bath of absolute ethanol and stained with cresyl fast violet. For each tissue sample, a second slide was stained with a conventional hematoxylin-eosin stain to assess tissue morphology. Total RNA was extracted using the RNeasy FFPE kit (Qiagen, Courtaboeuf, France). RNA yield and purity were assessed using a DS 11 spectrophotometer (Denovix Inc.). RNA (100 ng) was hybridized with a panel of selected probes (Nanostring Technologies, Inc., USA). After incubation at 65 °C, samples were processed on a Nanostring nCounter FLEX platform. Data analysis was performed using freely-available nSolver™ analysis software (version 4.0; NanoString Technologies, Inc.). The mRNA profiling data was normalized based on individual digital molecular barcodes to the expression levels determined for six specific housekeeping genes. Background signals that were estimated from blank wells were subtracted from the raw values. P-value adjustments were performed using the Benjamini-Hochberg false discovery rate (FDR) estimation method. Differentially expressed genes with a fold-change $\leq 2$ and $\geq 2$ and FDR-adjusted p-values $\leq 0.05$ were selected.

Cell culture

Normal human keratinocyte (NHK) cultures were established from abdominal skin samples from participants who were 20 (strain 1) and 32 (strain 2) years of age according to previously published procedures (Michopoulou et al., 2020). The cultured cells were grown in a keratinocyte growth medium supplemented with 0.15 mM $CaCl_2$ (KBM-2 BulletKit, Lonza Biosciences, Basel, Switzerland). To initiate experiments, $2\times10^5$ keratinocytes were cultured in CytoSoft® 6-well plates Elastic Moduli 2 kPa and 32 kPa (Sigma Aldrich) for 48 to 72 h in keratinocyte basal medium (KBM)-2. Wells were rinsed with sterile PBS and processed for RNA extraction. Cells were photographed using a Zeiss Axiovert 40 microscope equipped with a circular differential interference contrast and coupled to a Coolsnap Fx camera (Roper Scientific, Evry, France).

Statistical evaluation

Unpaired t-tests and one-tailed analysis of variance (ANOVA) were used to evaluate the data; a p-value < 0.05 was considered statistically significant. The size of the replicate measurements is indicated in the legend of each graph; the number of experimental repetitions is indicated by n. Individual data values are presented systematically for cases in which n < 6. Statistical analyses were performed using GraphPad Prism software, version 8.4.2 (GraphPad software). All results are presented as the mean $\pm$ standard error (SEM).

RESULTS

Dermal matrix stiffness restricts basal keratinocyte adhesion mechanisms

Because previous studies suggested that external mechanical stresses can influence rete ridge morphogenesis by activating keratinocyte proliferation (Topczewska et al., 2019; Wu et al., 2013), we performed a series of experiments designed to address the role played by the surrounding ECM. To address this question, we analyzed the stiffness of the ECM surrounding the rete ridges by atomic force microscopy (AFM). For this purpose, unfixed sections were prepared from frozen tissue blocks and used in experiments that examined force-volume AFM measurements, as previously described for the mechanical characterization of the ECM (Calò et al., 2020). Measurements were performed on biopsy specimens from three young and three older donors. Bright-field optical imaging facilitated the scanning of the location of dermal ECM areas immediately adjacent to the tops and bottoms of the rete ridges (Figure 1A) as well as the acquisition of $5 \times 5$ $\mu$m2 Force Volume (FV) maps (Figure 1B). All generated force-indentation curves were fitted with a standard Hertz model which permitted us to generate apparent elastic (E, Young's) modulus maps (Figure 1B). The mean E values determined for the biopsy specimens from donors who were 20, 20, and 26 years of age were significantly lower in the ECM at the top edges of the rete ridges (25 kPa $\pm$ 0.28) than at the bottom (35,6 kPa $\pm$ 0.36) (Figure 1C), which indicates the presence of a stiffness gradient along the rete ridges. As anticipated, and in agreement with previous reports, values for E measured in both areas of the dermal ECM were higher in specimens from older donors (those who were 51, 51, and 59

years of age). We even found that the mean E was greater at the upper edges of the rete ridge (55 kPa ± 0.93) than at the lower edges (42 kPa ± 0.86) in these specimens (Figure 1C). These data reveals that the increase of the ECM stiffness with aging is accompanied by reversal of the stiffness gradient along the rete ridges.

To determine whether stiffness has an impact on the expression of anchoring complex components, we cultured primary keratinocytes on stiff surfaces (Figure 2A) and examined their expression by polymerase chain reaction (PCR; Figure 2B). Two strains of human keratinocytes cultured on a low stiffness surface (2 kPa) remained round, convex, and slightly spread, whereas those cultured on a high stiffness surface (32 kPa) were spread out and frequently exhibited a differentiated phenotype (Figure 2A). Expression of the genes involved in HD formation, including encoding collagen XVII (*COL17A1*), LM-332 (*LAMA3*), plectin (*PLEC*), and BPAG1e (*DST*) was significantly reduced in cells plated on the rigid surface (Figure 2B and Figure 4A).

To assess the impact of changes in rete ridge shape with aging on the expression of genes involved in epidermal homeostasis, we performed microdissections on skin samples across the age spectrum. We extracted RNA and performed nanostring transcriptomic analyses with a keratinocyte-specific gene panel that included DEJ components, stem cell markers, mechanotransduction pathways, and proliferation markers to determine the changes in gene expression associated with aging. Of all the genes evaluated, significant changes in gene expression were observed only among those encoding components of the anchoring complexes (Figure 3). Specifically, we found higher levels of *COL7A1, COL17A1, ITGB4* and *LAMA3* transcripts in the epidermis in abdominal skin samples from young donors than in the foreskin samples and decreased levels in abdominal skin samples from the older donors. As anticipated, our results revealed that the level of *KRT15* transcripts decreased with donor age, regardless of their anatomical localization in the skin. By contrast, levels of *KLF4* gene transcript increased steadily (Figure 3). To confirm and reinforce the relevance of this latter finding, we examined the expression of immunoreactive KLF4 in the epidermis of abdominal skin samples from donors of increasing age (Figure 4A and Figure 4B). The ratio of KLF4 to DAPI staining underwent a regular and significant increase that paralleled the increasing age of the donors. We performed cell culture experiments (Figure 2A) and established a causal relationship between *KLF4* expression and the increasing stiffness of the ECM. We found a significant increase in *KLF4* transcription in keratinocytes cultured on a rigid support (Figure 5A and 5B). Taken together, our results indicated that the stiffness of the ECM has a profound influence on the HD-mediated adhesion of basal keratinocytes and promotes their proliferation and differentiation. The increase in stiffness observed in response to aging disrupts HD-mediated adhesion and accelerates the loss of progenitor cells.

EXAMPLE 2: Efficacy of molecules according to the invention to inhibits the activity of KLF4 :

Four molecules are tested for their efficacy to inhibit the activity of KLF4 : Calceolarioside B, Kuwanon E, Arctiin, Meso-secoisolariciresinol.

MATERIALS AND METHODS

The NHK cultures were created from foreskin and abdominal skin samples from female participants aged 36 and 44 years. The cultured cells were grown in a keratinocyte growth medium supplemented with 0.15 mM CaCl2 (KBM-2 BulletKit, Lonza Biosciences, Basel, Switzerland). At the beginning of the experiments, $3 \times 10^4$ keratinocytes were plated in two identical 96-well plates and cultured for 3 days. Treatment with the molecules or growth factor TGFα or Cacl2 was performed and left for 2 days at 37°C. One plate was processed for an XTT proliferation assay (Sigma-Aldrich, Saint-Quentin-Fallavier, France) the other plate was washed twice with PBS and the cells were fixed with 1% Paraformaldehyde. After washing twice with PBS, the cells were permeabilised with PBS/Triton 2% and then washed twice with PBS. A PBS/3% bovine serum albumin solution was added for 1 hour and the anti-KLF4 antibody (Millipore, Sigma-Aldrich) was applied for 1 hour. After washing three times with PBS, a solution of secondary antibody coupled to FITC was applied for 30 minutes. After washing the wells, the plates were read at a wavelength of 495 nm (excitation) / 519 nm (emission). Fluorescence intensity results were normalised with the cell viability data and expressed as a percentage of control cells treated with medium containing the corresponding amount of diluent.

RESULTS

All four molecules show a partial and dose-response inhibition of the activity of KLF4 (Figure 6). The strains derived from donors aged 36 and 44 years were selected for testing the molecules because their KLF4 expression level is higher in both quantitative immunodetection (Figure 6A) and RT-PCR (Figure 6B) compared to the keratinocytes isolated from the foreskin. Treatment of the three keratinocyte strains with TGFα (40 ng/ml) and 1.2 mM CaCl2 resulted in negative or positive regulation of KLF4 expression respectively, as shown by immunodetection of KLF4 in fixed cells (Figure 6C). The four molecules induced down regulation of KLF4 expression as compared to control cells after 48 hours of treatment, as demonstrated by immunodetection on cells (Figure 6D).

## Claims

1. Cosmetic non-therapeutic composition comprising at least one compound to inhibit the activity of KLF4.

2. The cosmetic non-therapeutic composition according to claim 1, for skin and/or scalp and/or hair and/or mucous membranes anti-aging, preferably for diminishing or preventing the consequences of stiffness of the extracellular matrix networks located at the epithelial-stromal interfaces of tissues of the skin and/or scalp and/or hair and/or mucous membranes.

3. The cosmetic non-therapeutic composition according to anyone of claims 1 and 2, in a cosmetically acceptable medium, at least one compound chosen among phenylpropanoids, flavonoids, lignans, phenols, benzophenone derivative, homoisoflavonoids, stilbenoids, alkaloids, diterpenoids, phloracetophenone glucoside, polyketides, diarylheptanoids and/or adlerol.

4. The cosmetic non-therapeutic composition according to anyone of claims 1 to 3, wherein said at least one compound is, in a cosmetically acceptable medium, at least one of Calceolarioside B, Arctiin, Meso-secoisolariciresinol, Kuwanon E, and/or Iriflophenone 3-c-beta-glucoside.

5. The cosmetic non-therapeutic composition according to claim 4, wherein the concentration of at least one of Calceolarioside B, Arctiin, Meso-secoisolariciresinol, Kuwanon E, and/or Iriflophenone 3-c-beta-glucoside in the composition is from 0.0001% to 10% by weight of the total composition.

6. The cosmetic non-therapeutic composition according to anyone of claims 1 to 3, wherein said at least one compound is, in a cosmetically acceptable medium, at least one of : Annullatin c, Phloracetophenone 4'-o-glucoside, Adlerol, Cristatic acid, 9'''-methyl salvianolate b, Parishin c, N-(2-methylaminobenzoyl)tryptamine, Salvianolic acid a, Dihydrocapsaicin, Silandrin, Angeflorin, 2-(3,4-dihydroxyphenyl)ethyl beta-d-glucopyranoside, 3-(3-hydroxy-1-oxo-2-phenylpropoxy)-2-phenylpropanoic acid, 1-(3,4-dihydroxyphenyl)-7-(4-hydroxyphenyl)-3,5-heptanediol, Itoside a, Leonurine, Uncinatone, (r)-3-(3,4-dihydroxybenzyl)-3,7-dihydroxy-4-chromanone, and/or Icariside h1.

7. The cosmetic non-therapeutic composition according to claim 6, wherein the concentration of at least one of Annullatin c, Phloracetophenone 4'-o-glucoside, Adlerol, Cristatic acid, 9'''-methyl salvianolate b, Parishin c, N-(2-methylaminobenzoyl)tryptamine, Salvianolic acid a, Dihydrocapsaicin, Silandrin, Angeflorin, 2-(3,4-dihydroxyphenyl)ethyl beta-d-glucopyranoside, 3-(3-hydroxy-1-oxo-2-phenylpropoxy)-2-phenylpropanoic acid, 1-(3,4-dihydroxyphenyl)-7-(4-hydroxyphenyl)-3,5-heptanediol, Itoside a, Leonurine, Uncinatone, (r)-3-(3,4-dihydroxybenzyl)-3,7-dihydroxy-4-chromanone, Icariside h1, in the composition is from 0.001% to 10% by weight of the total composition.

8. The cosmetic non-therapeutic composition according to anyone of claims 1 to 7, further comprising at least one cosmetically acceptable agent chosen from chosen from soothing agents, restructuring agents, regenerating agents, revitalizing agents, sunscreens, anti-wrinkle agents, moisturizing agents, anti-aging agents, surfactants, fats, organic solvents, solubilizing agents, thickening and gelling agents, smoothing agents, firmness-enhancing agents, elasticity and/or the barrier effect of the skin, antioxidants, opacifiers, thermal waters, mattifying agents, chemical or mineral filters, trace elements, stabilizing agents, foaming agents, perfumes, ionic or non-ionic emulsifiers, fillers, sequestrants and chelators, perfumes, filters, essential oils, coloring matters, pigments, hydrophilic or lipophilic active ingredients, lipid vesicles encapsulating one or more active ingredients and/or preservatives.

9. The cosmetic non-therapeutic composition according to anyone of claims 1 to 8, wherein said cosmetic composition is formulated in the form of a cream, an ointment, a mask, a serum, a milk, a lotion, a paste, a foam, an aerosol, a stick, a shampoo, a conditioner, patches, an aqueous-alcoholic or oily solution, an oil-in-water or water-in-oil or multiple emulsion, an aqueous or oily gel, a liquid, pasty or solid anhydrous product, a dispersion of oil in an aqueous phase using spherules, these spherules being polymeric nanoparticles such as nanospheres and nanocapsules or lipid vesicles of ionic and/or non-ionic type.

10. Non-therapeutic cosmetic process for skin and/or scalp and/or hair and/or mucous membranes anti-aging care, comprising the topical application of the composition according to any one of claims 1 to 9.

11. Use of a cosmetic non-therapeutic composition according to claim 1 to 9 to inhibit the activity of KLF4.

12. Use according to the preceding claim, for skin and/or scalp and/or hair and/or mucous membranes anti-aging care.

**13.** Use according to anyone of claims 11 and 12 for cosmetic non-therapeutic diminishing or preventing consequences of the stiffness of the extracellular matrix (ECM) networks located at the epithelial-stromal interfaces of tissues of the skin and/or scalp and/or hair and/or mucous membranes. ]

[Fig. 1]

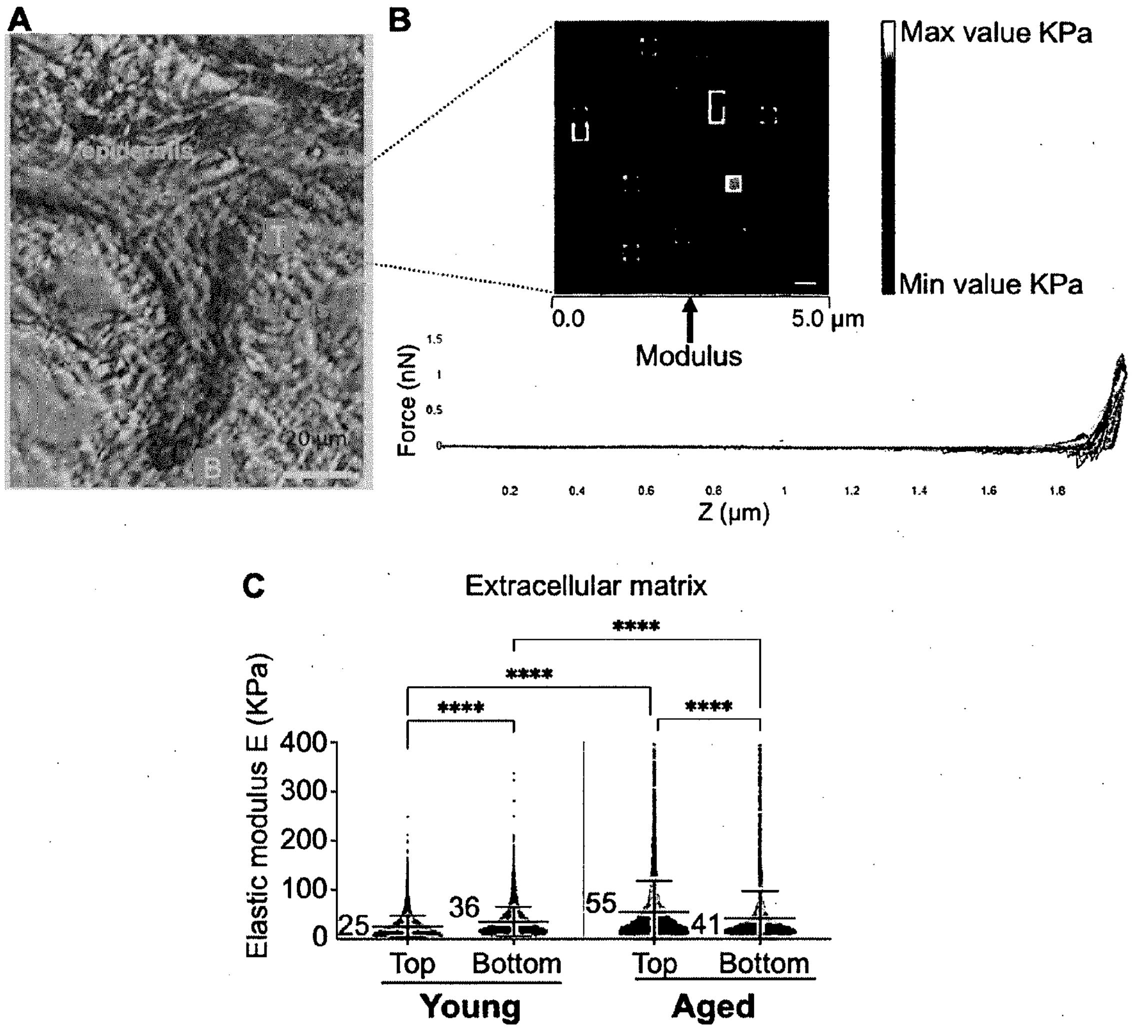
A
epidermis
T
B
20 µm
B
Max value KPa
Min value KPa
0.0
5.0 µm
Modulus
Force (nN)
1.5
1
0.5
0
0.2
0.4
0.6
0.8
1
1.2
1.4
1.6
1.8
Z (µm)
C
Extracellular matrix
****
****
****
****
Elastic modulus E (KPa)
400
300
200
100
0
25
36
55
41
Top
Bottom
Young
Top
Bottom
Aged

[Fig. 2]

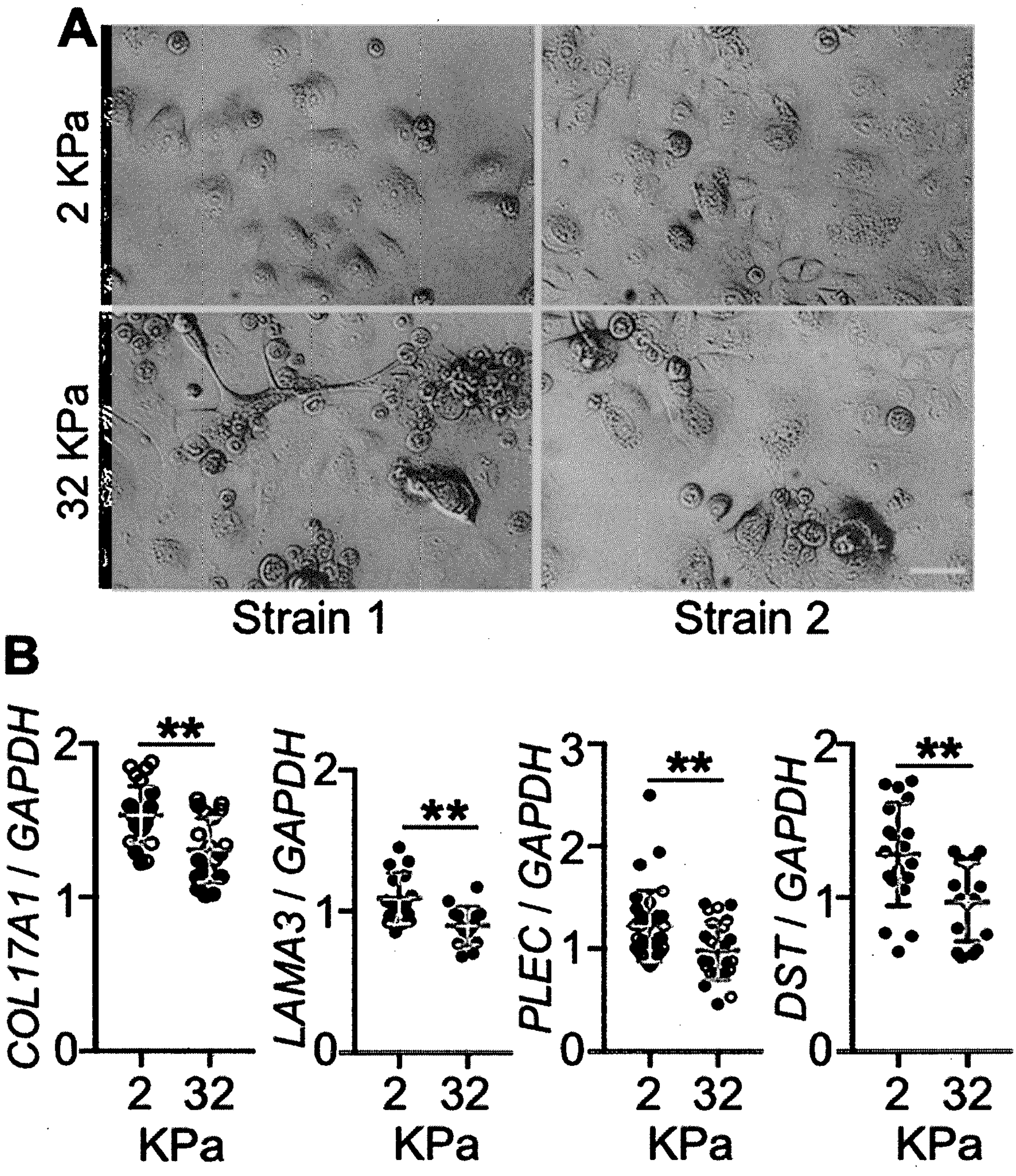
A
2 KPa
32 KPa
Strain 1
Strain 2
B
COL17A1 / GAPDH
LAMA3 / GAPDH
PLEC / GAPDH
DST / GAPDH
**
0
1
2
3
2 32
KPa

[Fig. 3]

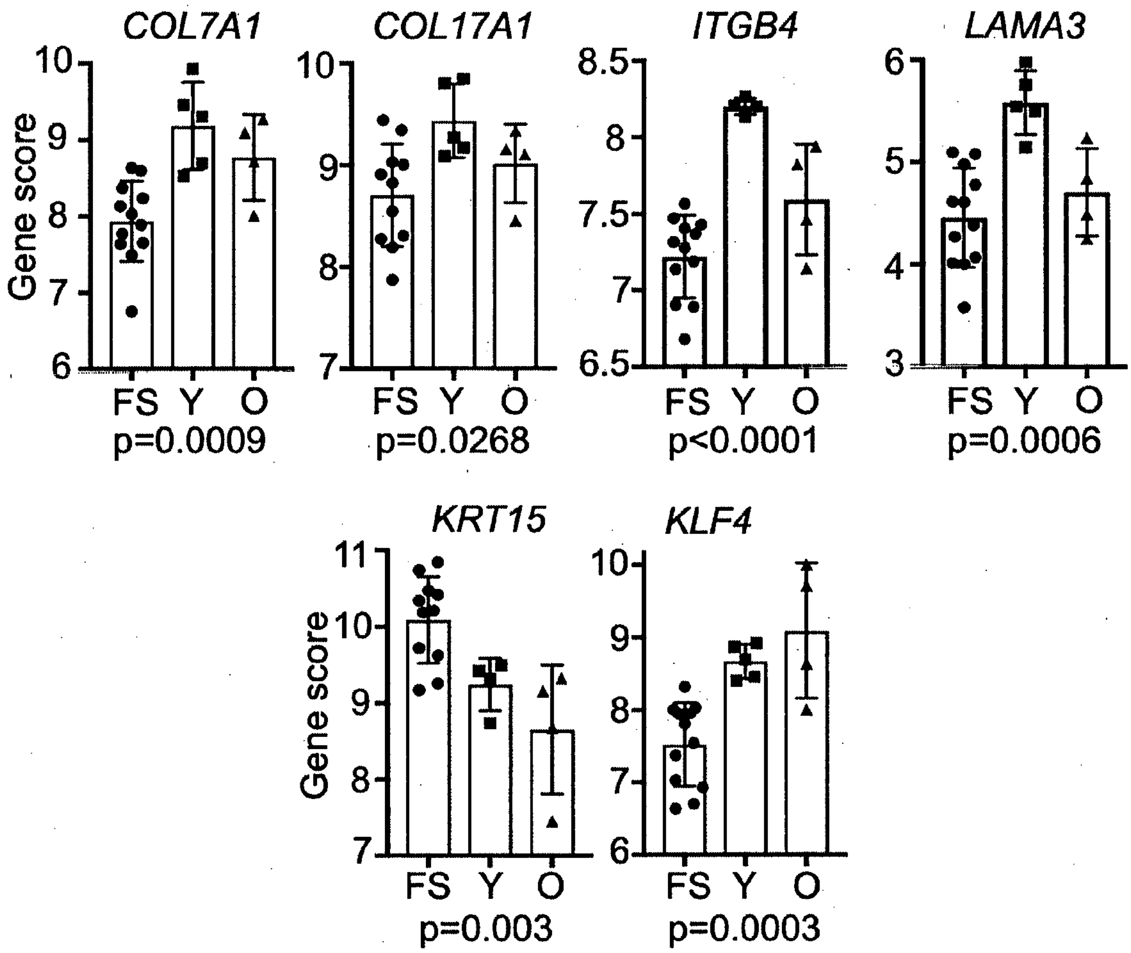
COL7A1
COL17A1
ITGB4
LAMA3
KRT15
KLF4
Gene score
FS Y O
p=0.0009
p=0.0268
p<0.0001
p=0.0006
p=0.003
p=0.0003

[Fig. 4]

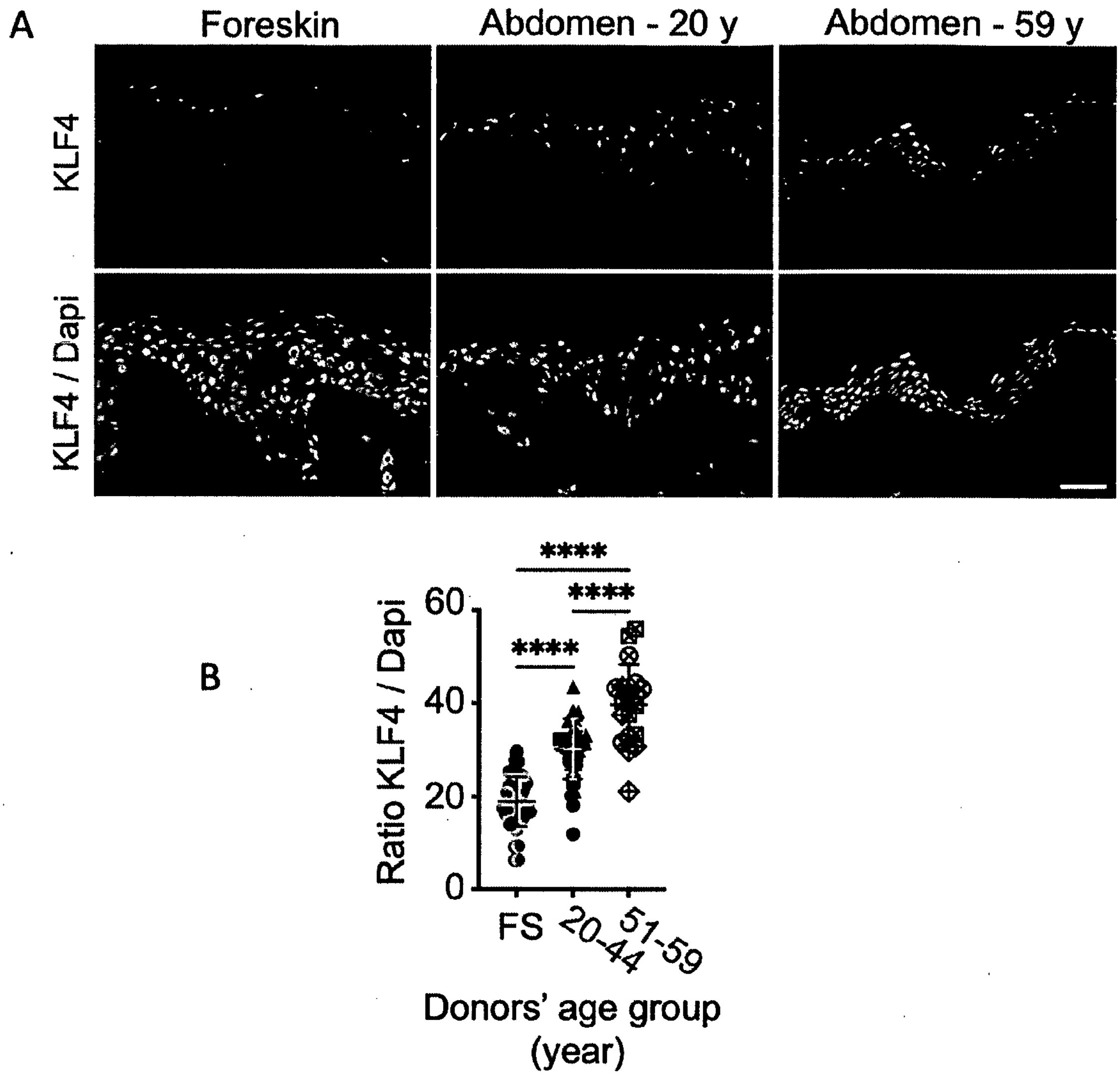
A
Foreskin
Abdomen - 20 y
Abdomen - 59 y
KLF4
KLF4 / Dapi
B
Ratio KLF4 / Dapi
****
****
****
60
40
20
0
FS
20-44
51-59
Donors' age group (year)

[Fig. 5]

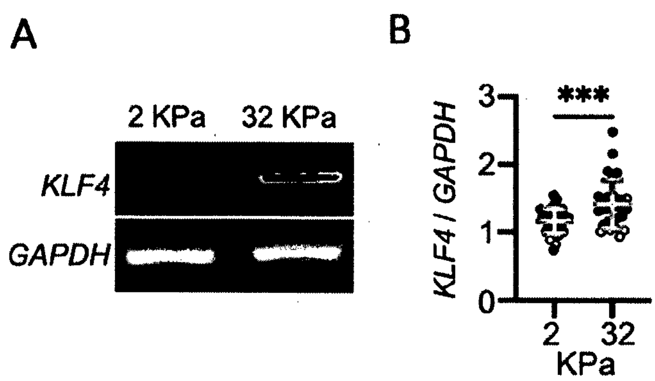
A
2 KPa
32 KPa
KLF4
GAPDH
B
KLF4 / GAPDH
***
3
2
1
0
2
32
KPa

[Fig. 6]

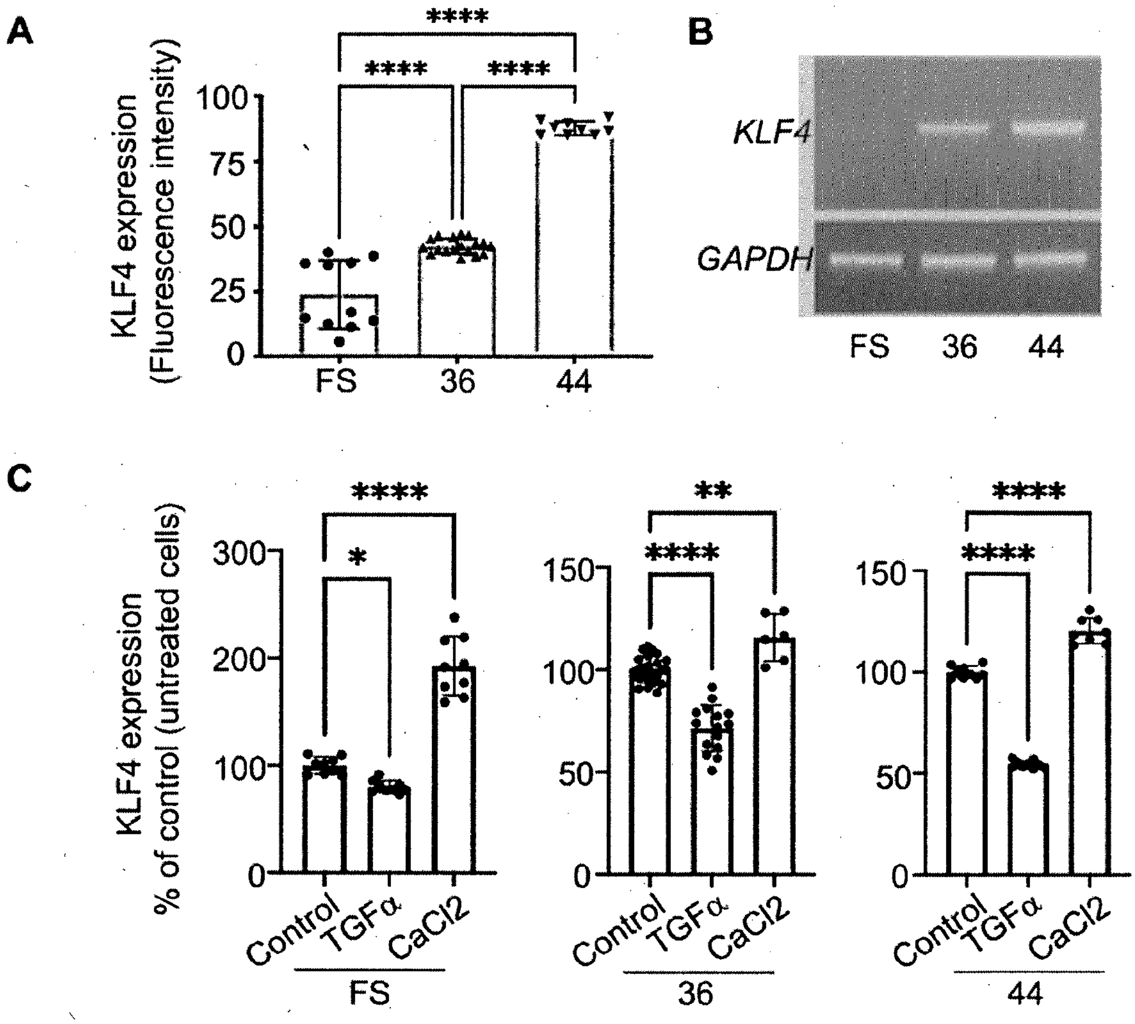
A
KLF4 expression
(Fluorescence intensity)
100
75
50
25
0
FS
36
44
****
B
KLF4
GAPDH
FS
36
44
C
KLF4 expression
% of control (untreated cells)
300
200
100
0
*
**
150
50
Control
TGFα
CaCl2

D

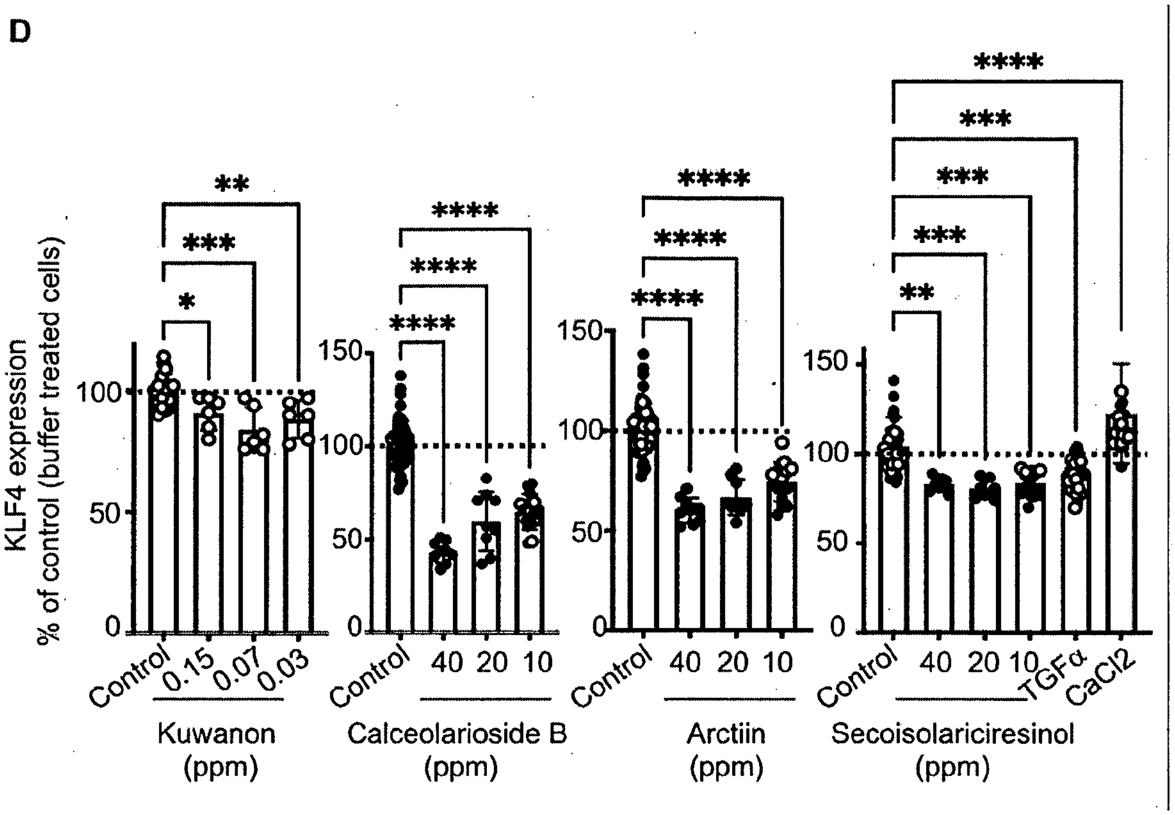
KLF4 expression
% of control (buffer treated cells)
0
50
100
150
*
***
**
****
***
Control
0.15
0.07
0.03
Kuwanon
(ppm)
Control
40
20
10
Calceolarioside B
(ppm)
Arctiin
(ppm)
Control
40
20
10
TGFα
CaCl2
Secoisolariciresinol
(ppm)

Europäisches Patentamt
European Patent Office
Office européen des brevets

# EUROPEAN SEARCH REPORT

Application Number
EP 24 31 5059

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2006/093633 A1 (STAB FRANZ [DE] ET AL) 4 May 2006 (2006-05-04)<br>* paragraphs [0025] - [0027], [0036], [0037] *<br>* claims 1-3, 6, 7, 11, 12 * | 1-5,8-10 | INV.<br>A61Q19/08<br>A61Q5/00<br>A61K8/34<br>A61K8/35<br>A61K8/365<br>A61K8/49<br>A61K8/9789 |
| X | KR 2018 0070854 A (LG HOUSEHOLD & HEALTH CARE LTD [KR]) 27 June 2018 (2018-06-27)<br>* paragraphs [0001], [0012], [0032], [0040], [0041] *<br>* claims 1, 2 * | 1-5,8-10 | |
| X | US 2022/142884 A1 (VERBAKEL JOOST [NL] ET AL) 12 May 2022 (2022-05-12)<br>* paragraphs [0002], [0035], [0036], [0039] *<br>* claims 1, 6, 9, 15 * | 1-3,6-10 | |
| X | DATABASE GNPD [Online]<br>MINTEL;<br>23 December 2008 (2008-12-23),<br>anonymous: "Eye & Lip Contour",<br>XP093189610,<br>Database accession no. 1025821<br>* the whole document * | 1-5,8-10 | **TECHNICAL FIELDS SEARCHED (IPC)**<br>A61Q<br>A61K |
| X | DATABASE GNPD [Online]<br>MINTEL;<br>22 May 2012 (2012-05-22),<br>anonymous: "Anti-Age Hand Cream",<br>XP093189611,<br>Database accession no. 1803483<br>* the whole document * | 1-5,8-10 | |
| A | US 2018/369115 A1 (ALMINANA DOMENECH NURIA [ES] ET AL) 27 December 2018 (2018-12-27)<br>* column 36, lines 16-19 * | 1-13 | |

-/--

The present search report has been drawn up for all claims

1

| Place of search | Date of completion of the search | Examiner |
|---|---|---|
| Munich | 30 July 2024 | Diebold, Alain |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 24 31 5059

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GHALEB AMR M ET AL: "Krüppel-like factor 4 (KLF4): What we currently know", GENE, ELSEVIER AMSTERDAM, NL, vol. 611, 22 February 2017 (2017-02-22), pages 27-37, XP029947049, ISSN: 0378-1119, DOI: 10.1016/J.GENE.2017.02.025 * the whole document * ----- | 1-13 | |

TECHNICAL FIELDS SEARCHED (IPC)

The present search report has been drawn up for all claims

1

| Place of search | Date of completion of the search | Examiner |
|---|---|---|
| Munich | 30 July 2024 | Diebold, Alain |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
......................................................................
& : member of the same patent family, corresponding document

EPO FORM 1503 03.82 (P04C01)

page 2 of 2

## ANNEX TO THE EUROPEAN SEARCH REPORT ON EUROPEAN PATENT APPLICATION NO. EP 24 31 5059

This annex lists the patent family members relating to the patent documents cited in the above-mentioned European search report. The members are as contained in the European Patent Office EDP file on
The European Patent Office is in no way liable for these particulars which are merely given for the purpose of information.

30-07-2024

| Patent document cited in search report | Publication date | Patent family member(s) | Publication date |
|---|---|---|---|
| US 2006093633 A1 | 04-05-2006 | DE 10223486 A1 | 11-12-2003 |
| | | EP 1511460 A1 | 09-03-2005 |
| | | US 2006093633 A1 | 04-05-2006 |
| | | WO 03099244 A1 | 04-12-2003 |
| KR 20180070854 A | 27-06-2018 | NONE | |
| US 2022142884 A1 | 12-05-2022 | CA 3149298 A1 | 04-02-2021 |
| | | EP 4003282 A1 | 01-06-2022 |
| | | ES 2962832 T3 | 21-03-2024 |
| | | US 2022142884 A1 | 12-05-2022 |
| | | WO 2021018935 A1 | 04-02-2021 |
| US 2018369115 A1 | 27-12-2018 | AU 2016366220 A1 | 28-06-2018 |
| | | BR 112018011643 A2 | 04-12-2018 |
| | | CN 108495646 A | 04-09-2018 |
| | | EP 3386528 A1 | 17-10-2018 |
| | | JP 7010823 B2 | 26-01-2022 |
| | | JP 2019502677 A | 31-01-2019 |
| | | KR 20180094008 A | 22-08-2018 |
| | | US 2018369115 A1 | 27-12-2018 |
| | | WO 2017100421 A1 | 15-06-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **HU D.** ; **CIE**. *Nat Commun*, September 2015, vol. 6, 8419
- *Cell Stress*, vol. 4 (8), 199-215